# FASCICULE DE BREVET EUROPEEN

(11) **EP 4 487 771 B1**
(45) Date de publication et mention de la délivrance du brevet: **18.03.2026**
(21) Numéro de dépôt: 24186428.9
(22) Date de dépôt: 04.07.2024
(51) Int. Cl.: A61B 5/145, A61B 5/1486

(54) **DISPOSITIF DE TYPE PATCH MICRO-AIGUILLES À BIOÉLECTRODE INTÉGRÉE**
MIKRONADELPFLASTERVORRICHTUNG MIT INTEGRIERTER BIOELEKTRODE
MICRONEEDLE PATCH DEVICE WITH INTEGRATED BIOELECTRODE

(30) Priorité: 06.07.2023 FR 2307216
(43) Date de publication de la demande: 08.01.2025
(73) Titulaire: Commissariat à l'Energie Atomique et aux Energies Alternatives, 75015 Paris (FR); Centre National de la Recherche Scientifique, 75016 Paris (FR); Université Grenoble Alpes, 38401 Saint-Martin-d'Hères (FR)
(72) Inventeur: DARMAU, Bastien, 38401 SAINT-MARTIN D'HERES (FR); GROSS, Andrew, 38610 GIERES (FR); TEXIER-NOGUES, Isabelle, 38054 GRENOBLE CEDEX 09 (FR)
(74) Mandataire: Ipsilon

(56) Documents cités:
- EP-A1- 4 201 966
- WO-A1-2018/115710
- WO-A1-2022/040177
- MENGJIA ZHENG ET AL: "Osmosis-Powered Hydrogel Microneedles for Microliters of Skin Interstitial Fluid Extraction within Minutes", ADVANCED HEALTHCARE MATERIALS, WILEY - V C H VERLAG GMBH & CO. KGAA, DE, vol. 9, no. 10, 30 April 2020 (2020-04-30), pages n/a, XP072469317, ISSN: 2192-2640, DOI: 10.1002/ADHM.201901683
- CALI� A ET AL: "Polymeric microneedles based enzymatic electrodes for electrochemical biosensing of glucose and lactic acid", SENSORS AND ACTUATORS B: CHEMICAL, ELSEVIER BV, NL, vol. 236, 1 June 2016 (2016-06-01), pages 343 - 349, XP029700070, ISSN: 0925-4005, DOI: 10.1016/J.SNB.2016.05.156
- LIU GUI-SHI ET AL: "Microneedles for transdermal diagnostics: Recent advances and new horizons", BIOMATERIALS, ELSEVIER, AMSTERDAM, NL, vol. 232, 26 December 2019 (2019-12-26), XP086001958, ISSN: 0142-9612, [retrieved on 20191226], DOI: 10.1016/J.BIOMATERIALS.2019.119740

## Description

### Domaine technique

La présente invention concerne le domaine des dispositifs utiles pour la caractérisation transdermique de biomarqueurs et analytes dans les fluides interstitiels. En particulier, un tel dispositif se présente sous la forme d'un patch micro-aiguilles couplé à une électrode comportant au moins une espèce biologiquement active, de préférence une enzyme et dites bioélectrode. Afin de limiter les prélèvements sanguins répétitifs, invasifs et douloureux, et d'apporter plus de confort aux patients, le développement de dispositifs transdermiques in/on-vivo a fait l'objet d'une attention considérable ces dernières décennies pour un suivi de la santé personnel portable et rapide. Ces dispositifs reposent sur la détection voire la quantification de biomarqueurs et analytes dans les fluides interstitiels (ISF) comme source adéquate d'informations biologiques pour mieux comprendre et gérer la santé humaine. L'ISF est un fluide corporel naturel présent entre les tissus et les cellules qui comprend des électrolytes, des nutriments et de nombreux autres biomarqueurs. L'ISF est également abondant dans le corps humain (environ 15 à 25 % du poids corporel humain), situé juste sous la peau dans les zones faiblement nerveuses telles que le derme ou l'hypoderme, et est donc facilement accessible. La composition en métabolites de l'ISF est souvent étroitement corrélée à celle du sang. Par exemple, pour la biodétection du glucose, le taux de glucose dans l'ISF est égal à environ 80 à 90 % de la concentration de glucose dans le sang (environ 5 mmol.l⁻¹). D'autres biomarqueurs et analytes présents dans l'ISF à des concentrations nmol.l-1 à mmol.L⁻¹ et pertinents pour les biocapteurs électrochimiques peuvent inclure, par exemple, le lactate, l'alcool, le nitrate, l'alanine, la cystéine, le pyruvate, le glycérol, le Ca²⁺, le Mg²⁺, le K⁺, le phosphate, l'urée, le cholestérol, le glutamate.

Les dispositifs portables de surveillance continue du glucose, dits encore CGM (*continuous glucose monitoring* en anglais), disponibles dans le commerce ont déjà révolutionné la surveillance et la gestion des niveaux de glucose dans l'ISF. Ces dispositifs électrochimiques reposent sur l'emploi d'une aiguille pour collecter l'ISF dans les tissus sous-cutanés. Les biocapteurs de glucose ampérométriques enzymatiques sont le type le plus courant de biocapteurs disponibles dans le commerce grâce à des avantages qui incluent la grande réactivité et la haute spécificité des enzymes pour les réactions enzymatiques et électroenzymatiques. Malgré une meilleure gestion du diabète, les inconvénients importants de ces dispositifs portables incluent la longueur de l'aiguille, qui est généralement comprise entre 5 et 7 mm et peut gêner les patients, et le capteur lui-même qui a une durée de vie, une exactitude et une précision limitées. Il existe également d'autres contraintes techniques, liées par exemple à la taille et à l'empreinte adhésive du dispositif, à la nécessité d'utiliser des membranes ou des médiateurs redox coûteux (composants biocatalytiques pour le biocapteur), ou encore à une biocompatibilité et une biodégradabilité limitées.

Les patchs microaiguilles, dits encore MN, sont devenus l'une des alternatives les plus prometteuses pour remplacer les aiguilles inconfortables ou invasives utilisées dans les dispositifs CGM. Les patchs MN sont des réseaux d'aiguilles de taille micrométrique avec des hauteurs généralement comprises entre 20 et 2000 µm qui sont capables de pénétrer spécifiquement la barrière cutanée pour atteindre la couche de derme et accéder à l'ISF dermique. Situé au-dessus de l'hypoderme, le derme est faiblement nerveux et vascularisé, ce qui rend les patchs transdermiques, MN, peu invasifs et moins douloureux, en raison de leurs dimensions réduites, exempts de sang et, et moins sujets à provoquer des infections. Néanmoins, la capacité d'offrir de tels avantages nécessite des considérations minutieuses dans la conception des patch MN liées à des facteurs tels que les matériaux utilisés, leur géométrie et la manière dont les microaiguilles sont appliquées.

Comme les CGM commerciaux de type aiguille, les biocapteurs intégrés dans ou sur un patch MN sont principalement basés sur des biocapteurs enzymatiques électrochimiques, en raison notamment de leur sélectivité, spécificité, faible coût et simplicité de fabrication. Les patchs MN sont généralement préparés par des procédés de micro-usinage, de lithographie, de gravure, d'impression et de micro-moulage, et sont fabriqués à l'aide de polymères et/ou de métaux et/ou de céramiques. Les différents types de MN sont les MN solides, creuses et poreuses.

Les MN solides à biocapteur intégré sont construites à base d'un support dur, par exemple polycarbonate, [Réf 1] Damien K Ming et al BMJ Innov, 2022, 8, 87 - 94, poly(méthyl méthacrylate) (PMMA), et la bioélectrode, à base d'un métal conducteur mince (par exemple Au, Pt) sur lequel sont déposés des composants biocatalytiques (enzyme, médiateur redox, etc., parfois inclus dans une matrice polymère poreuse) et parfois revêtus de couches polymères ou membranes biocompatibles pour des raisons de biocompatibilité et porosité. La bioélectrode est directement formée sur la surface externe des MNs qui comprend donc le support MN (en métal ou polymère rigide comme le polycarbonate) puis une couche avec les composants biocatalytiques (médiateur redox et enzyme), et éventuellement une membrane ou une couche en polymère biocompatible. De tels MN solides ont été utilisés pour la détection et/ou le suivi du glucose et d'autres métabolites. Malheureusement, le contact direct entre l'électrode du capteur et les tissus et le fluide biologique favorisent les problèmes de cytotoxicité et de stabilité. En outre, un délaminage des matériaux constituant la bioélectrode lors de l'insertion du dispositif MN dans la peau peut se produire (c'est-à-dire des dommages mécaniques à la couche d'électrode générant des débris métalliques résiduels laissés in vivo).

Pour les MN dites creuses, le capteur bioélectrode enzymatique est intégré soit (i) à l'intérieur du canal d'une aiguille creuse du patch MN [Réf 2] Hazhir Teymourian et al Anal. Chem., 2020, 92, 2, 2291 - 2300 soit (ii) à l'arrière du dispositif [Réf 3] US20090099427Al*.* Malgré des temps de détection généralement plus longs que pour le biocapteur à base de MNs solides, en raison de la force motrice (par exemple, les forces capillaires) nécessaire pour établir le contact entre le biocapteur et l'ISF/biomarqueurs, cette voie peut offrir une extraction de fluide efficace et une flexibilité en termes de type de bioélectrodes qui peuvent être incorporées. Néanmoins, la lixiviation des composants biocatalytiques dans l'ISF est toujours un problème. Il existe également des limitations en termes d'obtention d'une pénétration efficace car les aiguilles sont moins résistantes mécaniquement que des MNs pleines ni de géométrie idéale pour la pénétration cutanée.

Les MN en matériaux poreux (par exemple à base de poly(méthacrylate de glycidyle (PGMA) ou de soie) sont un type hybride entre les MN solides et creuses. [Réf 4] Hiroyuki Kai and al, J. Phys. Energy, 2021, 3, 024006*.* Les MN peuvent être considérées comme suffisamment solides pour garantir une pénétration efficace dans la peau, tout en fournissant une structure de pores, le cas échéant conductrice car métallique [Réf 4], adéquates pour (i) déposer sur les pores les enzymes et médiateurs constitutifs d'une bioélectrode et nécessaires à la détection électrochimique et (ii) améliorer le contact de l'ISF avec les enzymes et médiateurs et la surface conductrice constituant la bioélectrode d'un capteur. L'ISF peut être guidé vers le biocapteur via des forces capillaires comme c'est typiquement le cas pour les MN creuses.

Malheureusement, l'ensemble de ces biocapteurs MN-électrodes enzymatiques (bioélectrodes) intégrés ont pour inconvénient de requérir des processus de fabrication coûteux et longs et de n'être compatibles qu'avec des processus d'intégration d'électrodes complexes et souvent limités. Or, pour des raisons évidentes, ces méthodes d'intégration coûteuses et complexes sont un obstacle à leur production industrielle. Par ailleurs, tous les dispositifs MN pour lesquels les matériaux constituant l'électrode sont déposés sur la surface externe de la MN sont sujets à des problèmes de toxicité non bénins liés au capteur, par exemple, dans le cas où le revêtement se casse ou se décolle lors du perçage de la peau et/ou du fonctionnement de l'appareil, ou simplement en raison d'un contact direct entre le revêtement et les tissus et fluides cutanés. Certes, les MN de type creux ou poreux avec l'électrode à l'arrière des microaiguilles sont plus flexibles en termes de type d'électrodes pouvant être intégrées, et peuvent offrir une sécurité améliorée, mais, en revanche, ils requièrent un temps de latence accru et une extraction d'ISF contrainte. De plus, un problème courant est d'obtenir une bonne adhésion de l'électrode au dispositif MN. [Réf 7] SeungHyun Park et al Biosensors and Bioelectronics, 220, 2023, 1149121. D'autres dispositifs selon l'état de l'art sont décrits dans Mengjia Zheng et al: "Osmosis-Powered Hydrogel Microneedles for Microliters of Skin Interstitial Fluid Extraction within Minutes",Advanced Helathcare Materials, WILEY - V C H VERLAG GMBH & CO. KGAA, DE, vol. 9, no. 10, 30 avril 2020 et Lui Gui-Shi ET AL: "Microneedles for transdermal diagnostics: Recent advances and new horizons",Biomaterials, ELSEVIER, AMSTERDAM, NL, vol. 232, 26 décembre 2019 (2019-12-26).

En conséquence, il demeure un besoin d'un dispositif utile pour une mesure électrochimique transdermique, couplant au moins des microaiguilles avec au moins une bioélectrode, et qui soit de fabrication non complexe.

Il demeure également le besoin que la mise en œuvre de ce dispositif soit dénuée de risque de toxicité à l'égard du sujet faisant l'objet de l'analyse.

Il demeure également le besoin qu'un tel dispositif soit compatible avec la mise en œuvre d'une grande diversité de bioélectrodes.

Il demeure également un besoin qu'un tel dispositif procure une bonne adhésion de cette bioélectrode et qu'en particulier qu'elle n'y soit pas sujette à un phénomène de décollement.

Il demeure également un besoin que cette bioélectrode ait un contact rapide avec l'ISF à caractériser sans pour autant avoir un contact avec la peau.

La présente invention vise précisément à répondre à ces besoins.

### Résumé de l'invention

Ainsi, selon un de ses aspects, la présente invention concerne un dispositif utile pour des mesures électrochimiques transdermiques, ledit dispositif comportant au moins un support polymérique 1 ayant une surface dédiée à un contact avec une peau 2, au moins un réseau de micro-aiguilles polymériques 3 solidaires dudit support 1 et dépassant vers l'extérieur de ladite surface du support 1 dédiée au contact avec ladite peau 2, et au moins une bioélectrode 10 poreuse, le cas échéant nano- ou micro-structurée, et comprenant au moins une espèce biologiquement active, notamment une enzyme, immobilisée à la surface d'un matériau conducteur, caractérisé en ce que lesdites micro-aiguilles 3 et au moins la surface de contact dudit support 1 avec ladite peau 2 sont formées d'un hydrogel réticulé, biocompatible, non conducteur électronique à l'état sec et conducteur électrolytique au contact d'un fluide aqueux, et en ce que ladite bioélectrode 10 est disposée au contact de l'hydrogel dédié à gonfler au contact dudit fluide aqueux et est dénuée de contact direct avec la peau 2.

Au sens de l'invention, une bioélectrode poreuse est une bioélectrode apte à être, au moins en surface et le cas échéant en profondeur, interpénétrée par l'hydrogel.

Ainsi, selon un mode de réalisation particulier, une bioélectrode peut être formée d'un matériau conducteur qui est poreux notamment sélectionné parmi le groupe composé de platine, platine-iridium, or, palladium, iridium, leurs alliages, graphite, carbone, l'oxyde d'étain indium, dioxyde de ruthénium, nanotubes de carbone ou polymère conducteurs. Selon un autre mode de réalisation, une bioélectrode peut comprendre en surface de son matériau conducteur, et au contact de la ou les espèce(s) biologique(s) immobilisée(s), un ou des matériau(x) polymérique(s) poreux sélectionnés par exemple parmi le poly(oxyde d'éthylène), des polymères comportant des groupes azotés hétérocycliques tels que par exemple la poly(vinylpyridine) ou le poly(vinylimidazole), des polymères ionomères perfluorés tels que par exemple le Nafion, la poly(uréthane), des polymères redox (c'est-à-dire des polymères ayant de multiples centres redox basés sur des métaux de transition tel que l'osmium, un biopolymère polysaccharidique comme par exemple l'acétate de cellulose, le dextran ou le chitosan, un polymère conducteur à base de dérivés poly(aniline) ou poly(3,4-éthylènedioxythiophène), ou une de leurs combinaisons. Le matériau conducteur de la bioélectrode 10 peut donc, dans ce second mode de réalisation, être ou non, nativement poreux.

De manière inattendue, les inventeurs ont ainsi constaté que le couplage d'une bioélectrode, poreuse et le cas échéant nano-structurée ou micro-structurée, à un support comprenant au moins un réseau de micro-aiguilles peut être réalisé dans des conditions permettant de satisfaire aux attentes précitées sous réserve que ce support soit formé notamment d'un hydrogel conforme à l'invention, à savoir apte à évoluer d'un état non conducteur à l'égard des électrons lorsqu'il est à l'état sec vers un état conducteur électrolytique lorsqu'il est gonflé par un fluide aqueux. Un aspect poreux de la bioélectrode a en outre pour avantage de permettre la pénétration dudit hydrogel dans les pores de la bioélectrode et de favoriser ainsi le contact intime de la surface de la bioéléctrode sur laquelle est immobilisée au moins une espèce biologiquement active, de préférence une enzyme, le cas échéant en combinaison avec un médiateur électrochimique, avec le fluide interstitiel qui a imbibé l'hydrogel. Ce contact intime favorise l'accroche physique entre la bioelectrode et le polymère constitutif du substrat ou des aiguilles du patch MN, et favorise également les performances de l'espèce biologiquement active et de la bioélectrode.

Plus précisément, et comme il ressort des exemples ci-après, les micro-aiguilles 3 du dispositif selon l'invention possèdent avantageusement à l'état sec, la résistance mécanique nécessaire à la perforation d'un tissu corporel, notamment à des profondeurs comprises entre 200 et 2000 µm dans le cas d'une peau humaine, pour accéder à son fluide interstitiel, ISF, de façon efficace. Lorsque l'hydrogel réticulé et sec, constitutif de ces micro-aiguilles 3, se trouve au contact de ce fluide ISF, il se gonfle et le fluide à analyser va y diffuser par différence de pression osmotique et/ou capillarité. Ainsi, d'un point de vue électrique, les micro-aiguilles 3 de même que la partie du support 1 solidaire de ses microaiguilles 3, peuvent être considérées, lorsqu'elles sont imprégnées d'ISF, comme devenant une prolongation des tissus avec l'ISF et assurent alors une fonction d'électrolyte. La bioélectrode comprenant l'espèce biologiquement active disposée au contact de cet hydrogel gonflé d'ISF permet dans ces conditions de réaliser une mesure ou opération fidèle à celle qui serait réalisée si elle était insérée directement dans la peau mais avantageusement de façon minimalement invasive. De plus, la méthode d'intégration décrite dans l'invention permet de tirer profit de la porosité de la bioélectrode 10. En interpénétrant au niveau de la bioélectrode 10, l'hydrogel maximise la surface de contact entre la ou les espèces actives de la bioélectrode 10 et le fluide interstitiel véhiculé par l'hydrogel lors de son gonflement. Selon une variante de réalisation, la ou au moins une bioélectrode 10 est intégrée dans l'hydrogel réticulé constitutif du support 1 qui est en contact direct avec l'arrière des micro-aiguilles 3.

Selon une autre variante de réalisation, la ou au moins une bioélectrode 10 est intégrée dans l'hydrogel réticulé constitutif de micro-aiguille(s) 3.

En particulier, la bioélectrode 10 est au moins partiellement interpénétrée d'hydrogel réticulé.

Selon encore une autre variante de réalisation, le dispositif selon l'invention comporte au moins une électrode annexe, distincte ou non d'une bioélectrode. En particulier, le dispositif selon invention peut comprendre au moins une électrode annexe distincte d'une bioélectrode notamment choisie parmi une électrode de référence, une contre-électrode, une électrode hybride de référence et une contre-électrode.

Dans un tel mode de réalisation, le dispositif selon l'invention peut comprendre au moins l'une des électrodes, en particulier une bioélectrode 10 poreuse, dans l'hydrogel réticulé constitutif du support 1 en contact direct avec l'arrière des micro-aiguilles 3 et au moins une voire plusieurs autres électrodes dans l'hydrogel réticulé constitutif de micro-aiguille(s) 3.

Le dispositif selon l'invention est donc avantageux à plusieurs titres.

Son hydrogel réticulé constitutif est compatible avec l'intégration de n'importe quelle bioélectrode 10 pour la détection des analytes, ions et/ou biomarqueurs. Cet hydrogel procure également à une bioélectrode enzymatique un environnement aqueux approprié, pour fonctionner dans des conditions satisfaisantes et permet en particulier de prolonger sa durée de vie/stabilité.

Seul son composant hydrogel réticulé est en contact direct avec le tissu biologique faisant l'objet de l'analyse, et donc le risque de délaminage du revêtement et/ou matériau constitutif de la bioélectrode (par exemple, médiateur, enzyme, couche AgCl, etc...), lors de l'insertion des micro-aiguilles dans le tissu est écarté.

Le risque de détachement de composant(s) de la bioélectrode 10 est également écarté compte-tenu de l'intégration de celle-ci en profondeur de l'hydrogel réticulé du dispositif selon l'invention.

Le positionnement de la bioélectrode 10 près de l'arrière des pointes des micro-aiguilles 3 et/ou dans des micro-aiguilles 3 elles-mêmes, limite également la distance que l'analyte, ion, proton et/ou biomarqueur doit parcourir et/ou toute limitation de transport de masse qui pourrait affecter les performances de détection y compris la réduction du temps de latence. Selon une variante particulière, le dispositif comprend au moins un bioélectrode 10 comprenant une enzyme notamment une oxydoréductase.

En particulier, la bioélectrode 10 est de type biocapteur électrochimique de glucose notamment de type 2^{ième} génération qui associe l'enzyme glucose déshydrogénase flavine adénine dinucléotide-dépendante (FAD GDH) à un médiateur redox, la 1,10-phénantholine-5,6-dione (PLQ), adsorbé physiquement au niveau d'une électrode poreuse en nanotubes de carbone multiparois (MWCNT) notamment tel que décrit dans le document WO2018 115710A1.

Selon une variante particulière, le dispositif selon l'invention se présente sous la forme d'un patch transdermique micro-aiguilles, MN. En particulier, les micro-aiguilles polymériques (3) ne sont pas creuses.

Selon une variante préférée, l'ensemble support 1 et micro-aiguilles 3 est constitué dudit hydrogel réticulé, biocompatible et non conducteur électronique à l'état sec.

En particulier, l'hydrogel réticulé est dénué de constituant métallique et de polymère conducteur électronique.

Selon une autre variante préférée, l'hydrogel est dénué d'espèce biologiquement active autre que celle(s) immobilisée(s) sur la ou lesdites bioélectrode(s) 10.

Selon une autre variante préférée, ladite espèce biologiquement active est une enzyme, notamment une oxydoréductase, et participe dans une réaction électrocatalytique avec un échange d'électrons entre l'enzyme et le matériau conducteur.

Selon un autre de ses aspects, la présente invention concerne un procédé de préparation d'un dispositif selon l'invention, notamment par micromoulage, et comprenant au moins la réticulation d'au moins un polymère, de préférence un biopolymère, pour former ledit hydrogel réticulé caractérisé en ce qu'au moins ladite ou une bioélectrode 10 poreuse et le cas échéant nano-ou micro-nanostructurée est intégrée par mise en contact avec ledit polymère avant ou simultanément à sa réticulation.

En particulier, ladite bioélectrode 10 voire les électrodes annexes sont disposées au contact de la formulation aqueuse contenant au moins ledit polymère non réticulé et y subissent une pression mécanique pour les positionner en profondeur à proximité de la base des micro-aiguilles 3 et/ou en profondeur de micro-aiguille(s) 3, préalablement ou simultanément à la réticulation.

Selon encore un autre de ses aspects, la présente invention concerne l'utilisation d'un dispositif selon l'invention pour des mesures électrochimiques transdermiques, en particulier pour la caractérisation d'au moins un analyte dans un liquide interstitiel.

Ainsi, l'invention vise tout particulièrement, un procédé de détection et/ou de dosage d'au moins un analyte dans un liquide interstitiel d'un patient, notamment d'une manière non-invasive, comprenant au moins la mise en contact des micro-aiguilles 3 d'un dispositif selon l'invention avec ledit liquide interstitiel dudit patient dans des conditions propices au gonflement dudit hydrogel réticulé constitutif des micro-aiguilles 3 par ce liquide et à la diffusion de ce liquide par différence de pression osmotique et/ou capillarité jusqu'à la ou les bioélectrode(s) 10 dudit dispositif.

D'autres caractéristiques, variantes et avantages des objets de l'invention, ressortiront mieux à la lecture de la description, des exemples et figures qui vont suivre, donnés à titre illustratif et non limitatif de l'invention.

Dans la suite du texte, les expressions « compris entre ... et ... », « allant de ... à ... » et « variant de ... à ... » sont équivalentes et entendent signifier que les bornes sont incluses, sauf mention contraire.

### Brève description des dessins

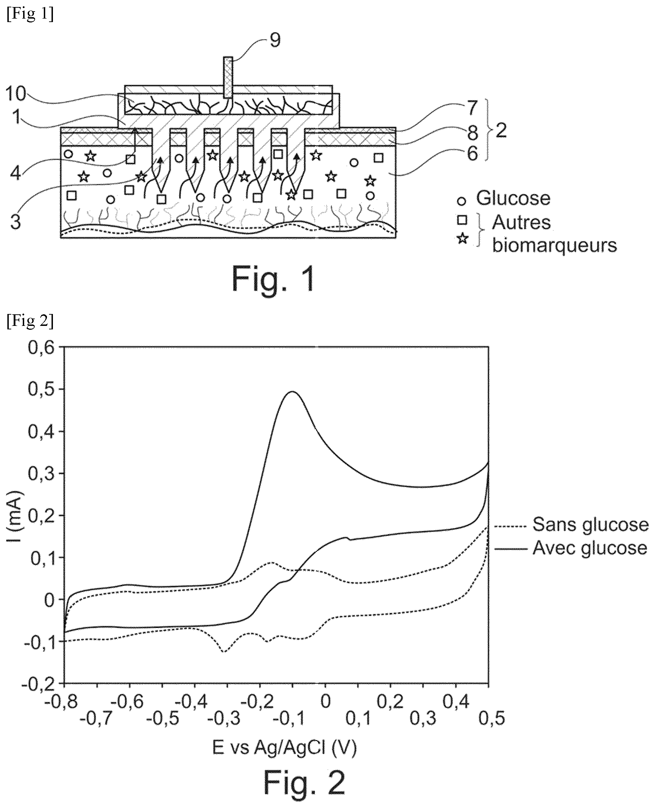

[Fig 1] représente un exemple de schéma de dispositif selon l'invention.
[Fig 2] représente des voltammogrammes cycliques enregistrés à une vitesse de balayage = 1 mV.s-1 dans le tampon phosphate (PB) (0,1 M, pH = 7,4) avec un biocapteur à micro-aiguilles en dextran-méthacrylate (Dex-MA) avec un degré de substitution (DS = 9%) réticulé comprenant une électrode à base de MWCNTs recouverts de PLQ et FADGDH immobilisés (MN-BS) en absence (ligne pointillée) de glucose et en présence (trait plein) de glucose 0,1 M.

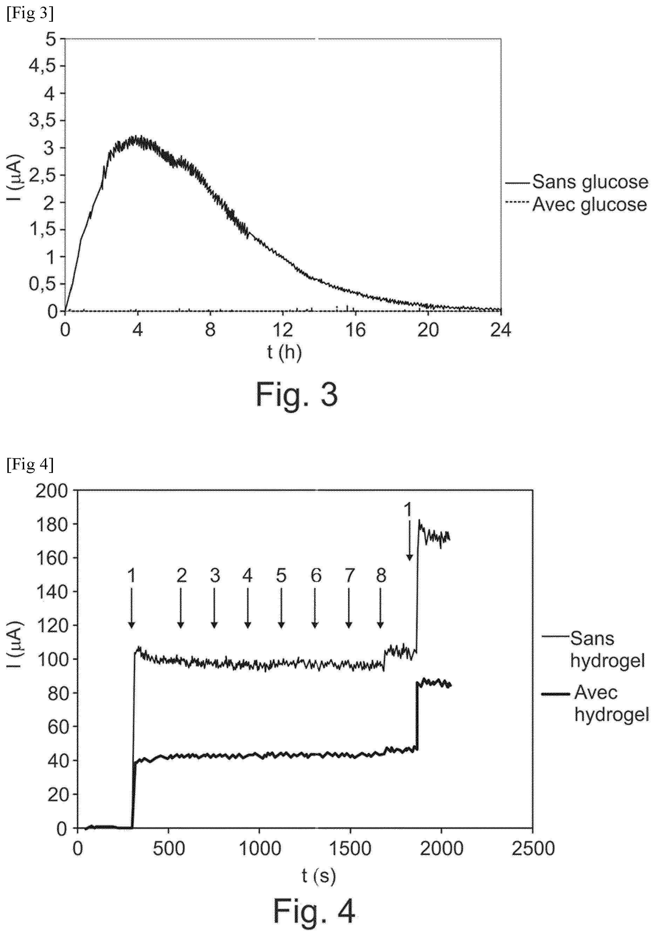

[Fig 3] illustre la surveillance continue de la glycémie par chronoampérométrie à -0,1 V vs pseudo-référence Ag/AgCl avec un biocapteur à micro-aiguilles en Dex-MA réticulé (DS = 37 %) comprenant une électrode à base de MWCNTs recouverts de PLQ et FADGDH immobilisés (MN-BS) dans une peau artificielle contenant 5 mmol.L-1 de glucose, n = 1.
[Fig 4] rend compte d'un test de sélectivité par chronoampérométrie dans du PB à -0,1 V vs pseudo-référence Ag/AgCl et avec une agitation de 300 tr/min avec un biocapteur à base de MWCNT sans revêtement hydrogel et avec revêtement hydrogel en présence de 5 mmol.L-1 de glucose (1), puis ajouts successifs de 2) 0,2 mmol.L-1 d'acétaminophène, 3) 1 mmol.L-1 de cholestérol , 4) 8 mmol.L-1 d'urée, 5) 2 mmol.L-1 de lactate, 6) 0,3 mmol.L-1 de galactose, 7) 0,5 mmol.L-1 d'acide urique UA, 8) 0,1 mmol.L-1 ascorbique.

### Description détaillée

Comme il ressort de ce qui précède, l'invention vise à proposer des dispositifs peu invasifs pouvant intervenir transdermiquement, en continu ou non, pour la détection transdermique de biomarqueurs dans les fluides interstitiels.

Plus particulièrement, ce dispositif comprend au moins un support polymérique 1, au moins une bioélectrode 10, poreuse et des micro-aiguilles 3 disposées sur l'une des faces du support 1. Ces micro-aiguilles 3 pénètrent dans un tissu biologique jusqu'au liquide interstitiel de celui-ci et dans lequel il est précisément cherché à caractériser électrochimiquement au moins une espèce chimique ou biologique.

Dans le dispositif selon l'invention, les micro-aiguilles 3 sont pleines. En d'autres termes, elles ne sont pas creuses mais constituées d'hydrogel réticulé conforme à l'invention et, le cas échéant, d'une bioélectrode 10 intégrée dans cet hydrogel mais sans contact direct possible avec le tissu faisant l'objet de l'analyse.

Elles ont généralement une longueur variant de 200 à 3000 µm.

Dans une variante de réalisation, le dispositif selon l'invention peut comprendre plusieurs réseaux de micro-aiguilles 3 et/ou plusieurs bioélectrodes 10 et/ou électrodes distinctes. En particulier, le dispositif selon l'invention se présente sous la forme d'un patch micro-aiguilles, MN, notamment tel qu'illustré en figure 1.

Le dispositif en figure 1 est formé d'un corps fait d'un hydrogel réticulé qui est, hors utilisation, à l'état sec. Le corps comporte un support 1 et des microaiguilles 3 qui font saillie d'une face 4 du support 1 en contact avec la peau 2 d'un patient et qui ont pénétré dans le derme 6 de la peau du patient, à travers la couche cornée 7 et l'épiderme 8.

Le dispositif comporte en outre une bioélectrode 10. Cette bioélectrode 10 est poreuse ce qui fait que l'hydrogel peut interpénétrer la structure de l'électrode (comme représenté sur la Fig 1).

Le fluide interstitiel présent dans le derme comprenant les biomarqueurs diffuse grâce à l'hydrogel vers la bioélectrode 10 à travers les microaiguilles 3 puis dans le support 1, et enfin dans les pores de la bioélectrode poreuse 10, en formant un hydrogel réticulé gorgé d'électrolyte (fluide interstitiel).

Un raccordement électrique 9 de la bioélectrode, peut être réalisé par un fil en un matériau électriquement conducteur (par exemple un fil plaqué argent cuivre) adhérant au dos de la bioélectrode avec une pâte conductrice (tel que de la pâte de carbone).

### Hydrogel réticulé

Au sens de l'invention, un hydrogel réticulé est un polymère tridimensionnel hydrophile et insoluble. Sa réticulation est obtenue par des liaisons physiques ou chimiques, de préférence chimiques, existantes entre des chaînes polymériques.

A l'état sec, l'hydrogel réticulé possède la dureté, nécessaire à la pénétration des micro-aiguilles 3 qu'il compose mais en revanche n'est pas apte à assurer la circulation d'électrons. En d'autres termes, il n'est pas conducteur électronique. Il est ainsi dénué de constituant métallique et de polymère conducteur organique à l'image par exemple du PEDOT, du poly(pyrrole) ou de la poly(aniline).

Par ailleurs, cet hydrogel réticulé et sec est capable de se gonfler au contact d'un fluide, notamment un fluide aqueux comme de l'eau ou un fluide biologique. Il peut donc absorber et retenir une grande quantité d'un fluide. Ce taux de gonflement de l'hydrogel (SR) est généralement défini par le rapport entre le poids de l'hydrogel gonflé et le poids de ce même hydrogel à l'état sec correspondant. Plus précisément, le fluide diffuse à l'intérieur de l'hydrogel réticulé par des phénomènes de capillarité et/ou de différence de pression osmotique entre le fluide et l'hydrogel. Ainsi imprégné, l'hydrogel réticulé rend possible la mise en contact du fluide aqueux à caractériser avec notamment la bioélectrode qu'il intègre. En particulier, l'hydrogel en pénétrant au moins partiellement la bioélectrode poreuse accroit la surface de contact entre le fluide et la bioélectrode. En d'autres termes, le gonflement de l'hydrogel par le liquide interstitiel fait que le dispositif selon l'invention, isolant à l'état sec, devient une matrice électrolytique (conducteur ionique), et permet de faire le pont électrique entre le tissu où sont insérées les micro-aiguilles 3 et la ou les bioélectrodes et, éventuellement électrodes, qu'il contient. Par opposition à des dispositifs de l'art antérieur, l'hydrogel réticulé et gonflé selon l'invention agit en outre comme un filtre isolant la ou les bioélectrodes/électrodes, qu'il intègre, de tout contact possible avec le tissu biologique. Le réseau structural du gel réticulé est tel qu'à l'état gonflé il rend quasi impossible toute migration d'éventuels petits fragments de la ou les bioélectrodes/électrodes vers ce tissu. Tout risque de toxicité du fait des bioélectrodes/électrodes est donc exclu.

L'hydrogel réticulé selon l'invention est également biocompatible.

Au sens de l'invention, un hydrogel biocompatible est un hydrogel qui du fait de sa nature chimique n'est pas de nature à provoquer un effet indésirable au niveau du tissu biologique faisant l'objet de l'analyse.

L'aspect biocompatible de l'hydrogel est un atout car seul lui est en contact direct avec les tissus en tant que composant des micro-aiguilles 3 pénétrant dans la peau d'un sujet. Ainsi, l'hydrogel constitutif des microaiguilles 3 et du support 1 joue le rôle d'écran entre le tissu et la bioélectrode 10 et des éventuelles électrodes annexes qui lui sont associées dans le dispositif selon l'invention. De plus, si l'hydrogel bien que réticulé reste biorésorbable sur le long terme, les composants hydrogel des micro-aiguilles 3 en polymère ne soulèveront aucun risque en cas de casse d'une micro-aiguille dans la peau 2 pendant l'utilisation du dispositif, puisque qu'ils pourront être résorbés au cours du temps sans dommage pour les tissus.

Enfin, compte-tenu de sa biocompatibilité, l'hydrogel peut jouer un rôle bénéfique à l'égard de la stabilité de bioélectrode(s) 10 associée(s) dans le dispositif selon l'invention. Il constitue un environnement biocompatible notamment très avantageux pour les enzymes, permet de réduire leur dénaturation et peut permettre un transport de masse favorable. Comme déjà précisé, l'hydrogel réticulé selon l'invention possède avantageusement de bonnes propriétés de gonflement (gonflement en 24h supérieur à 10%, de préférence à 20%, plus préférentiellement de plus de 50%) et à l'état sec une bonne dureté mécanique pour transpercer la peau (module d'Young supérieur à 10 kPa).

Comme détaillé ci-après, le polymère précurseur de cet hydrogel doit être également à l'état "liquide" pour l'intégration d'au moins une bioélectrode 10 voire des électrodes annexes et rendre possible sa pénétration au moins partiellement dans la structure de ladite bioélectrode. La solution de polymère peut aussi être solide à température ambiante mais liquide à une température compatible avec la ou les espèces biologiquement actives, notamment la ou les enzymes, associée(s) à la bioélectrode (par exemple <60 degrés ou éventuellement <80 degrés pour les enzymes thermostables d'ingénierie). C'est le cas par exemple des gels de gélatine.

L'hydrogel réticulé selon l'invention peut être obtenu par réticulation de tout type de polymère qui, une fois réticulé, forme en contact avec un fluide aqueux un hydrogel insoluble et infusible.

En particulier, l'hydrogel réticulé selon l'invention peut être obtenu par réticulation d'un ou plusieurs biopolymères avec le cas échéant d'un ou plusieurs polymères synthétiques, le cas échéant modifiés chimiquement pour être réticulables, ou d'un de leurs mélanges.

Au sens de l'invention, le terme "biopolymère" désigne un polymère dérivant de ressources naturelles renouvelables (un matériau dit encore "naturel). Les biopolymères considérés selon l'invention ont l'avantage de posséder, outre une très bonne compatibilité avec la peau humaine, une biodégradabilité en tant que tels voire in vivo.

Ce terme « biopolymère » couvre ainsi les polymères naturels tels que par exemple une protéine ou un polysaccharide mais également les dérivés de polymères naturels comme par exemple ceux qui dérivent d'une modification chimique de ces polymères naturels pour les rendre réticulables ou compatibles avec un mode de réticulation spécifique.

Cette modification chimique consiste le plus souvent à introduire dans la structure des polymères à modifier des motifs réticulables en particulier choisis parmi les motifs thiol, divinylsulfonyle, maléimide, azoture, alcynyle, alcényle, acrylate, méthacrylate, aldéhyde, norbornènyle, oxy-amine, et en particulier parmi les acrylates, méthacrylates.

A titre illustratif de polymères synthétiques convenant pour former un hydrogel réticulé convenant à l'invention peuvent notamment être cités les poly(éthylène glycol) (PEG) bruts ou modifiés par différentes fonctions chimiques comme par exemple les poly(éthylène glycol) diacrylique (PEGDA), poly(éthylène glycol)diméthacrylique (PEGDMA) les poly(vinyl pyrrolidone) (PVP), les poly(methyl vinyl ether/acide maléique-PEG, les PVA-MA, -PLA, -PLLA, -PGA, -PLGA et leurs dérivés.

A titre illustratif de biopolymères convenant pour former un hydrogel réticulé conforme à l'invention, peuvent notamment être cités les polyhydroxyacides, tels que par exemple l'acide polyglycolique (PGA) et l'acide polylactique (PLA), également connu sous le nom de polylactide ; les polysaccharides, tels que par exemple la cellulose, la chitine et l'amidon et des protéines ou des peptides, tels que par exemple le collagène, la gélatine, les élastines, les fibroïnes, la zéine de maïs, la soie de diverses espèces de vers à soie, la kératine et leurs dérivés

Selon une variante de réalisation préférée, l'hydrogel réticulé et biocompatible considéré selon l'invention est obtenu par réticulation d'au moins un biopolymère choisi parmi les polymères de type chitosane, dextran, alginates, collagène, gélatine, agarose, chitine, polyhydroxyalcanoates, pullane, amidon, amylose, amylopectine, cellulosique en particulier carboxyméthylcellulose, et hydroxypropylcellulose, acide hyaluronique, gomme gellane, gomme xanthane comme par exemple les dérivés de ces polymères issus de leur modification chimique pour les rendre réticulables ou compatibles pour un mode de réticulation spécifique et leurs combinaisons.

Selon un mode de réalisation particulier, ce biopolymère est ou dérive d'au moins un polymère choisi parmi les alginates, l'acide hyaluronique, la carboxyméthylcellulose, le chitosan, le dextran et leurs dérivés.

En particulier, l'hydrogel est un hydrogel réticulé de dextran.

Au sens de l'invention, le terme dextran couvre les dextrans ayant été ou non chimiquement modifiés pour être réticulés.

Ainsi, il peut être intéressant de réticuler un polymère de dextran chimiquement modifié par des motifs acrylates et/ou méthacrylates qui sont bien connus pour être photopolymérisables. Ces groupements fonctionnels qui rendent possible une réticulation par irradiation, sont introduits avec un degré de substitution donné, DS. Conventionnellement, le DS correspond au nombre de fonction polymérisable introduit sur 100 unités monomères. Ce DS est déterminable par 1H RMN.

Ainsi selon un mode de réalisation particulier, le dispositif selon l'invention comprend un hydrogel dérivant de la réticulation d'un polymère de dextran, modifié par des motifs choisis parmi les acrylate, méthacrylate, alcènyle, et alcynyle, de préférence un polymère de dextran modifié par des motifs méthacrylate, Dex-MA, et de DS variant de 5 à 65%.

### Electrodes et leur mode d'intégration

Une autre spécificité du dispositif de l'invention repose sur le mode d'intégration de la ou des bioélectrode(s), poreuse(s) requise(s) selon l'invention voire d'autre(s) électrodes annexes.

Comme précisé ci-dessus, les électrodes sont disposées de manière à ne pas avoir de contact direct avec le tissu dont l'ISF fait l'objet de l'analyse.

D'une manière générale, elles sont entièrement intégrées dans l'hydrogel réticulé devant être gonflé par cet ISF, à l'exception de leur extrémité dédiée au raccordement électrique externe.

Généralement ce raccordement est localisé sur la face supérieure ou l'une des faces latérales du dispositif.

Par opposition à des dispositifs classiques, la ou le bioélectrode(s) et éventuellement électrode(s) annexe(s) ne sont également pas dans le cadre de la présente invention en communication avec un canal auquel seraient reliées les micro-aiguilles.

Comme déjà précisé ci-dessus, elles sont en revanche disposées soit à proximité et/ou au sein de micro-aiguilles pleines du dispositif.

Selon un mode de réalisation particulier, elles sont intégrées dans l'épaisseur du support localisé à l'arrière des micro-aiguilles.

Ce mode d'intégration est notamment illustré en figure 1.

### Bioélectrodes

Les bioélectrodes sont des électrodes largement décrites dans la littérature et l'homme du métier est à même grâce à ses connaissances générales d'accéder à des bioélectrodes satisfaisant aux spécificités de l'invention. Le terme « bioélectrode » fait très souvent référence à une électrode comportant une ou plusieurs enzyme(s).

En particulier, une bioélectrode selon l'invention peut comprendre un matériau conducteur choisi parmi les électrodes à base de Pt, Au, Ag, Pd, Ni, Ir, carbone graphitique, carbone amorphe, graphène, oxyde de graphène, diamant, diamant dopé au bore, nanotubes, fibres dopées semi-conductrices ex. silicium dopé, oxydes métalliques, par ex. oxyde d'indium-étain, électrodes polymères conductrices par exemple un matériau PEDOT, et des fibres conductrices.

Une bioélectrode peut être nano-structurées ou micro-structurées. Il peut ainsi s'agir d'une nano- ou micro-électrode.

Sa forme générale peut être plane ou non. Une bioélectrode plane peut être une électrode imprimée sur un substrat poreux ou non. Ce substrat peut comprendre une feuille de papier carbone ou de tissu ou une feuille ou une pastille ou une électrode imprimée, par ex. électrode sérigraphiée.

Une bioélectrode de forme non plane comprend un fil de Pt ou un fil de fibre de carbone ou une électrode enroulée.

Par exemple, les bioélectrodes convenant à l'invention peuvent être choisies parmi les bioélectrodes à base de Pt, Au, Ag, Pd, Ni, Ir, carbone graphitique, carbone amorphe, graphène, oxyde de graphène, diamant, diamant dopé au bore, nanotubes, fibres dopées sémi-conductrices ex. silicium dopé, oxydes métalliques, par ex. oxyde d'indium-étain, électrodes polymères conductrices e.g. PEDOT et des fibres conductrices.

Lorsque le matériau conducteur de la bioélectrode selon l'invention est non poreux, il est modifié en surface par un matériau poreux polymérique ou inorganique. Ce matériau peut notamment être choisi parmi des hydrogels réticulé ou des polymères comme par exemple le Nafion, un acétate de cellulose, un poly (ether cétone) sulfoné, du glutaraldéhyde, un poly(éthylène glycol) diglycidyl ether, poly(dimethyl siloxane), du chitosan, un dextran, un alginate, des polymères redox basés sur des composés et complexes de métaux de transition, du poly(oxyde d'éthylène), des polymères comportantdes groupes azotés hétérocycliques tels que la poly(vinylpyridine) ou le poly(vinylimidazole), des poly(uréthane), des polymères conducteurs à base de dérivés poly(aniline) ou de poly(3,4-éthylènedioxythiophène), et leurs combinaisons ? ou copolymères.

Il est à noter qu'un tel matériau est également avantageux pour améliorer les performances de la bioélectrode en termes de stabilité ou capacité de détection de molécules ou espèces interférentes. Il est donc possible de le considérer en association avec un matériau conducteur poreux.

Selon une autre variante, les bioélectrodes 10 considérées selon l'invention sont formées d'un matériau conducteur nano-structuré ou micro-structuré.

Il peut ainsi s'agir de bioélectrodes poreuses 3D telles que les « papiers » constitués de nanotubes de carbone et de bioélectrodes micro/nanostructurées 2D.

En particulier, la bioélectrode est à base de nanotubes de carbone et en particulier à base de nanotubes de carbone multiparois (MWCNT).

Au moins une espèce biologiquement active, notamment une enzyme, est immobilisée à la surface du matériau conducteur.

Avantageusement, la bioélectrode comprend au moins une enzyme, le cas échéant combinée à une molécule qui facilite le transfert des électrons entre l'enzyme et l'électrode 10, comme par exemple un médiateur électrochimique ou une molécule de type 'promoteur' qui améliore l'orientation et/ou le transfert des électrons entre l'électrode et l'enzyme.

En revanche, l'hydrogel dudit dispositif est avantageusement dénué d'espèce biologiquement active ou de médiateur, autre que celle(s) immobilisée(s) sur la ou lesdites bioélectrode(s).

Ainsi, le dispositif peut intégrer une bioélectrode sur laquelle est ou sont immobilisée(s), généralement par adsorption, une ou plusieurs enzymes notamment choisies parmi les oxydases, par ex. glucose oxydase, pyruvate oxydase, xanthine oxydase, lactate oxydase, les déshydrogénases par ex. lactate déshydrogénase, les réductases par ex. nitrate et nitrite réductase et les métalloenzymes, par ex. bilirubine oxydase ou laccase, etc.

Une bioélectrode peut ainsi figurer une électrode de travail, WE. Le terme « électrode de travail » ici désigne une électrode à laquelle un composé candidat (analyte, biomolécule, agent actif) est électro-oxydé ou électro-réduit avec ou sans l'intervention d'un médiateur redox ou molécule 'promoteur'. Le terme « médiateur redox » désigne un agent de transfert d'électrons qui transfère des électrons entre un composé et une électrode de manière directe ou indirecte. Le terme « promoteur » désigne un agent qui facilite l'orientation des enzymes à la surface et/ou le transfert des électrons entre le site actif de l'enzyme et l'électrode. Dans un mode de réalisation particulier, le dispositif selon l'invention comprend une bioélectrode enzymatique.

La bioélectrode peut être également modifiée avec un catalyseur biologique (ou non) pour améliorer ses performances, par ex. sélectivité, spécificité, activité.

Selon encore une autre variante, le dispositif peut intégrer une bioélectrode sur laquelle est immobilisé un catalyseur non enzymatique tel que par exemple des nanoparticules conductrices, par ex. Au, Pt, Ir. et/ou autres matériaux nanostructurés, par ex. les nanotubes de carbone, les boîtes quantiques, du carbone mésoporeux et dopé, et/ou des molécules redox telles que les molécules organiques ou les espèces organométalliques.

Selon encore une autre variante, la bioélectrode selon l'invention peut être également modifiée par un élément de bioreconnaissance, par ex. stretavadine, extravaïdine, aptamères, etc.

De telles bioélectrodes sont particulièrement intéressantes pour détecter et/ou doser un analyte chimique ou biologique comme par exemple des protéines, des acides aminés, virus, hormones, des médicaments, des drogues (par exemple, les cannabinoïdes, les amphétamines, la cocaïne et les opioïdes) et les métabolites de la nicotine (par exemple la cotinine), et en particulier un analyte choisi parmi les glucose, lactate, alcool, nitrate, alanine, cystéine, pyruvate, glycérol, ions Ca^{2+,} Mg²⁺, K⁺, phosphate, urée, cholestérol , glutamate, peroxyde d'hydrogène, hydrogène, etc. Ce type de bioélectrode est souvent à base de métal ou carbone et est utilisée pour l'électro-oxydation ou électro-réduction du composé candidat et souvent avec un ou plusieurs éléments catalytiques ou électrocatalytiques comme des métaux nobles, des nanoparticules, et des enzymes. Il peut en particulier s'agir d'électrodes de WE avec des éléments de reconnaissance (souvent dites 'récepteurs') pour faciliter la détection spécifique et sélective et souvent avec un élément polymérique pour faciliter des propriétés comme les propriétés mécaniques, de reconnaissance et/ou de transmission de signal.

Ainsi et comme déjà précisé ci-dessus, un dispositif selon l'invention comprend avantageusement au moins une bioélectrode enzymatique et en particulier dédiée à interagir avec le glucose et qui associe l'enzyme FAD-GDH au médiateur redox phénanthroline quinone (PLQ) adsorbé physiquement au niveau d'une électrode en nanotubes de carbone multiparois (MWCNT) notamment telle que décrite dans le document WO2018 115710A1. Dans une autre exemple un dispositif selon l'invention peut comprendre au moins une bioélectrode avec une enzyme immobilisée et au moins une espèce redox immobilisée sélectionnée parmi un composé d'osmium, de ruthénium, de fer et de cobalt, couplée à un polymère sélectionné parmi du poly (vinylpyridine), de la poly(aniline), du poly(thiophène), du poly(acétylène), du poly(pyrrole), un biopolymère à base de poly(saccharides), ou parmi des molécules aromatiques choisies dans le groupe formé par la 9,10- phénanthrènequinone, la 1,10-phénanthroline-5,6-dione, la 9,10-anthraquinone, la phénanthrène, la 1 ,10-phénanthroline, la 5-méthyl-1,10-phénanthroline, les phénazines, les phénathiozines, tétrathiofulvalène, ou des complexes inorganiques parmi un composé d'osmium, de ruthénium, de fer et de cobalt, ou des polyoxométallates. L'espèce redox immobilisé servira à améliorer la réaction électrocatalytique ou bioélectrocatalytique.

Bien entendu, un dispositif selon l'invention peut intégrer plusieurs bioélectrodes qui sont respectivement aptes à interagir avec des espèces biologiques dits encore biomarqueurs, distinctes comme par exemples le lactate et le glucose. L'intégration de plusieurs bioélectrodes à capteurs différents dans un même dispositif a pour avantage de rendre possible par exemple la surveillance simultanée et en temps réel de plusieurs biomarqueurs dans les fluides interstitiels.

Dans une autre variante de réalisation, une seconde bioélectrode dédiée à la même cible, peut servir pour une mesure en duplicat ou à titre d'électrode de calibration.

Dans une variante, le dispositif selon l'invention comporte au moins une électrode annexe, distincte de la bioélectrode, notamment choisie parmi une électrode de référence, une contre-électrode, et une électrode hybride de référence et contre-électrode. Le capteur fonctionne alors de sorte qu'un courant est généré entre l'électrode de travail figurée par la bioélectrode et la contre-électrode ou l'électrode hybride.

Ainsi, le dispositif selon l'invention peut en outre comprendre une contre-électrode, CE. De telles électrodes sont particulièrement intéressantes pour établir un circuit avec une électrode de travail sur lequel le courant est appliqué ou mesuré. Les électrodes de Pt, Ag, AgCl, ou autres métaux (Ti, Au, Pd, étain), oxydes métalliques (IrO₂), électrodes de carbone (carbone vitreux), électrodes en polymères conducteurs (PEDOT), sont des électrodes auxiliaires typiquement utilisées en électrochimie.

Le dispositif selon l'invention peut également comprendre au moins une électrode de référence, RE. De telles électrodes sont particulièrement intéressantes pour la mesure ou le contrôle du potentiel d'un indicateur ou de l'électrode de travail. Les électrodes au calomel saturé ou électrode au chlorure d'argent sont des électrodes de référence typiquement utilisées en électrochimie.

Le dispositif selon l'invention peut également comporter un dispositif de détection électrochimique, attaché à ladite ou auxdites électrodes via des connections électriques préférentiellement entourées d'isolant. Ce dispositif de détection permet ainsi de réaliser des mesures par potentiométrie, voltamétrie cyclique (CV), voltamétrie cyclique à balayage rapide (FSCV), voltamétrie à onde carrée (SWV), voltampérométrie pulsée ou chronoampérométrie.

### Procédé de préparation d'un dispositif selon l'invention

Comme précisé ci-dessus, l'invention vise également un procédé de préparation d'un dispositif selon l'invention, notamment par micromoulage, comprenant au moins une étape de réticulation d'au moins un polymère, en particulier un biopolymère, pour former ledit hydrogel réticulé caractérisé en ce que ladite bioélectrode dudit dispositif est intégrée par mise en contact avec ledit polymère avant ou simultanément à sa réticulation.

En particulier, lors de la mise en contact de la bioélectrode voire des électrodes annexes, le polymère se présente sous la forme d'une formulation, notamment aqueuse, liquide ou semi-liquide c'est-à-dire d'aspect visqueux et de préférence présentant une viscosité variant de 1 à 100 Pa.s⁻¹ à 1 Hz avec une contrainte de cisaillement de 0,2 %.

Cette formulation liquide, de préférence aqueuse, contient ledit polymère et de préférence au moins un agent réticulant.

La ou les électrodes à intégrer sont disposées en surface de cette formulation liquide et y subissent une pression mécanique pour les positionner en profondeur à proximité de la base des micro-aiguilles ou au sein de l'hydrogel constitutif de microaiguilles, préalablement ou simultanément à la réticulation. En effet, à cette viscosité, la/les électrode(s) avec leur prise de contact peu(ven)t être insérée(s) aisément avec une force modérée (pression légère du pouce par exemple) de façon précise et/ou reproductible. Elles peuvent être insérées de façon que les fils de reprise de contact (ou d'autres contacts électriques) sortent préférentiellement du patch par le haut et/ou le côté du support solidaire des microaiguilles.

De plus, l'aspect liquide de la formulation de polymère au moment de l'insertion de la bioélectrode poreuse permet au polymère de pénétrer dans la structure de l'électrode et d'ainsi obtenir une grande surface de contact avec l'hydrogel qui véhiculera le fluide interstitiel.

Après insertion de la/les électrode(s), la réticulation est réalisée ou poursuivie jusqu'à l'obtention d'un hydrogel réticulé et sec. Pour ce faire, l'opération de réticulation est généralement suivie d'une opération de séchage.

La réticulation peut être une réticulation chimique. En quel cas, un réticulant est également présent dans la formulation liquide contenant le ou les polymère(s) et de préférence le ou les biopolymère(s) à réticuler. Ce mode de réticulation souvent lent est propice à l'intégration de la ou des électrodes au cours de la réticulation.

La réticulation peut être également déclenchée à l'aide d'un stimulus physique comme notamment la chaleur, une exposition gazeuse ou une irradiation (par exemple, lumière, rayonnement UV, rayons X, rayonnement gamma, irradiation micro-ondes).

Selon un mode de réalisation particulier, la réticulation est réalisée par voie photochimique.

D'une manière avantageuse, les dispositifs selon l'invention peuvent être préparés par une technique de micromoulage utilisant un moule en silicone, lui-même obtenu à partir d'un modèle maître réalisé par micro-usinage.

La formulation liquide, de préférence aqueuse, contenant le biopolymère et de préférence au moins un agent réticulant est versé dans le moule obtenu à partir de ce modèle maître. Ce moule permet d'obtenir après évaporation de l'eau ou du solvant de la solution, et réticulation, le dispositif en hydrogel réticulé sec qui peut être démoulé.

### Utilisation

Un dispositif selon l'invention est avantageusement utile pour des mesures électrochimiques transdermiques, en particulier pour la caractérisation d'au moins un analyte dans un liquide interstitiel.

Au sens de l'invention, le terme analyte couvre toute espèce chimique ou biologique comme un médicament, un agent bioactif, un métabolite ou encore un produit biochimique endogène.

N'importe quel analyte présent dans le liquide interstitiel cutané peut être caractérisé en utilisant le dispositif selon l'invention. Les exemples incluent, mais sans s'y limiter, le glucose, le sodium, le potassium, l'alcool, le lactate (important pour les athlètes), le cortisol, l'urée, les drogues (par exemple, les cannabinoïdes, les amphétamines, la cocaïne et les opioïdes) et les métabolites de la nicotine (par exemple la cotinine) ainsi que les substances médicamenteuses qu'un patient peut prendre pour une ou plusieurs conditions médicales.

Ainsi, la présente invention vise également un procédé de détection et/ou de dosage d'au moins un analyte dans un liquide interstitiel, notamment d'une manière non invasive, comprenant au moins la mise en contact des micro-aiguilles d'un dispositif selon l'invention, avec ledit liquide interstitiel dans des conditions propices au gonflement dudit hydrogel constitutif des micro-aiguilles par ce liquide et à la diffusion de ce liquide par différence de pression osmotique et/ou capillarité jusqu'à la bioélectrode dudit dispositif.

Les exemples et figures qui suivent sont présentés à titre illustratif et non limitatif du domaine de l'invention.

### Matériels

Dextran T70 (Dex T70, Mw = 70 000 g/mol) et Dextran T20 (Dex T20, Mw = 20 000 g/mol) de Pharmacosmos,
Anhydride méthacrylique (94%),
Lithium phenyl-2,4,6-trimethylbenzoylphosphinate (LAP, ≥ 95%),
Tampon phosphate (PBS 10X) : 1370 mM NaCl, 27 mM KCl, 100 mM Na2HPO4, 18 mM KH₂PO₄ ; pH = 7,4),
NaH₂PO₄ (≥ 99%),
D-glucose (≥ 99%),
1,10-phénantholine-5,6-dione (PLQ, 97%),
N,N-dimethylformamide (DMF, 99.9%), gélatine de porc (gel strength 300, Type A), agar, bovine serum albumine (BSA, ≥ 95%) de chez Sigma-Aldrich,
MWCNTs, (Ø = 9.5 nm, 1.5 µm longueur, ≥ 95% pureté) de chez Nanocyl,
Filtres PTFE de Millipore (JHWP, 0.45 µm de taille de pore, Ø (filtre) = 46 mm), polydimethylsiloxane et son agent réticulant (PDMS, Sylgard 184) de chez Dow,
Enzyme flavine adenine dinucleotide-dependent glucose dehydrogénase (FAD GDH) de chez Sekisui
Tampons phosphate (PB 0,1M Na₂HPO₄ ; pH = 7,4) et PBS (PBS1X: 137 mM NaCl, 2.7 mM KCl, 1 mM Na₂HPO₄, 1.8 mM KH₂PO₄ ; pH = 7,4)

**Fluide interstitiel artificiel (ISF)** : il a été préparé en dissolvant 22 g.L⁻¹ de BSA dans le tampon PBS et en ajustant le pH à 7,4. Les solutions de glucose dans le PB, le PBS et l'ISF ont été préparées à 1M en solubilisant la quantité adéquate de glucose dans le PB, PBS ou ISF et en ajustant le pH à 7.4.

**Derme artificiel (AD) :** il a été préparé comme suit : plusieurs solutions de gélatine (4 % ou 24 % (p/p)) et d'agar (1 % (p/p)) ont été mélangées dans 40 mL d'ISF artificiel en présence et en l'absence de glucose (5 mmol.L^{- 1}) dans un flacon de 100 mL, et chauffées à 80°C pendant 30 minutes sous agitation. Ensuite, la solution (AD 4 ou AD24, comprenant respectivement 4 ou 24 % de gélatine) a été coulée dans des boîtes de Pétri et laissée refroidir pendant une nuit avant utilisation.

**Fantôme de peau :** il est composé d'une fine membrane en téflon (filtre Millipore PTFE, JHWP, taille des pores 0,45 µm, Ø (filtre) = 47 mm) pour mimer la couche supérieure de l'épiderme (imite la couche cornée sèche), et du derme artificiel décrit ci-dessus préparé dans du PBS avec 22 g.L-1 de BSA en présence ou absence de glucose 5 mmol.L⁻¹ pour mimer la couche inférieure du derme.

Les mesures électrochimiques ont été effectuées à l'aide d'un multipotentiostat Biologique VMP3 avec le logiciel EC-lab ou d'un Princeton Applied research PARSTAT MC (PMC 1000/DC) exécutant le logiciel Versa Studio avec un système à trois électrodes composé de
- une pseudo-électrode de référence en fil Ag/AgCl maison (sat. KCl),
- un fil de platine comme contre-électrode et
- une électrode en carbone vitreux modifié ou une électrode à base de MWCNT modifiée ou le dispositif MN-BS intégré décrit ici comme électrode de travail.

Les mesures ampérométriques ont été réalisées dans une cage de Faraday à température ambiante à l'aide d'un potentiostat Princeton Applied research PARSTAT MC (PMC 1000/DC) exécutant le logiciel Versa Studio avec un système à trois électrodes, composé d'un fil en Ag/AgCl comme pseudo-électrode de référence, d'un fil de platine comme contre-électrode et de l'électrode de travail sélectionnée.

### Exemple 1

### Préparation de dextran modifié chimiquement

Des dextrans méthacrylés ont été synthétisés selon la méthode décrite dans la demande FR 2 114492.

Le protocole détaillé est comme suit : du dextran (5 g) a été dissous dans 100 mL d'eau distillée, DW, dans un bécher. Après dissolution complète, différents équivalents d'anhydride méthacrylique de 0,0625 à 0,5 équivalent par rapport aux fonctions hydroxyles du dextran ont été ajoutés goutte à goutte dans la solution de polymère afin d'obtenir le degré de substitution (DS) souhaité pour le polymère, soit 9, 18, 37 ou 62%. Ensuite, le pH a été ajusté à l'aide de NaOH (3 mol.l-1) pour être autour de 9 - 11 pendant toute la réaction. La solution a été agitée à température ambiante pendant 1 h. Enfin, le Dextran modifié a été dialysé contre de l'eau distillée pendant 1 semaine (membrane 12-14 kDa) et lyophilisé. Le solide blanc a été stocké à -20°C avant utilisation.

Une solution de PBS contenant 20 % (p/p) de Dex-MA avec un DS de 9, 18, 37 ou 62 % et 1 % (p/p) de LAP comme photoinitiateur, a été préparée dans un flacon de 10 ml. 100 µl de solution ont été versés dans un moule en Téflon cylindrique de 6 mm de diamètre (sans cavité micro-aiguilles) et la solution a été laissée sécher à l'air pendant 24h. Le polymère a été réticulé par une première irradiation du dos du patch à 405 nm pendant 1 min (P = 75 mW/cm2), suivie du démoulage du cylindre de polymère sec. Sont ainsi obtenus 4 matériaux de dextran-méthacylate (DS de 9, 18, 37 ou 62 %) photo-réticulés secs.

Ces 4 matériaux ont été caractérisés par un test de gonflement.

Pour ce faire, les 4 matériaux Dex-MA photo-réticulés séchés ont été pesés (poids WO). Les échantillons ont été placés dans un flacon contenant du PBS (1X) à température ambiante (21-24°C). Ils ont été retirés toutes les heures et leur surface a été rapidement essuyée avec une serviette en papier pour éliminer l'excès d'eau. Ensuite, les échantillons ont été immédiatement pesés (Wt) et replacés dans du PBS pour les points de mesure suivants. Cette opération a été répétée jusqu'à ce qu'aucun autre changement de poids ne soit détecté. Le taux de gonflement (SR) a été calculé selon SR = (Wt - WO) / WO. Trois expériences ont été réalisées en parallèle pour chaque matériau.

Le tableau 1 ci-après rapporte les valeurs moyennes des taux de gonflement ainsi déterminés.

**[Tableau 1]**

| **Echantillon Dex-MA** | **Taux de gonflement (%)** |
|---|---|
| **DS=62%** | 43.9 ± 2.2 |
| **DS=37%** | 55.2 ± 2.0 |
| **DS=18%** | 84.2 ± 1.5 |
| **DS=9%** | 98.0 ± 2.5 |

Le taux de gonflement maximum pour chaque formulation a été atteint en environ 1-2 heures. Une augmentation du SR est corrélée avec une diminution du DS.

La dureté de chacun de ces 4 matériaux a également été caractérisée par un test de compression.

Ces tests de compression ont été réalisés à l'aide d'un texturomètre TAXT.Plus (Stable Micro Systems, UK).

La vitesse de compression a été fixée à 0,5 mm.s-1 avec une force de compression maximale allant jusqu'à 50 N. Les valeurs de module de compression, E, ont été calculées comme la pente de la courbe de la force appliquée en fonction du déplacement. Le tableau 2 rend compte de ces valeurs.

**[Tableau 2]**

| **Echantillon** | **Module de compression (MPa)** |
|---|---|
| Dex-MA DS=62% | 159 ± 7 |
| Dex-MA DS=37% | 167 ± 10 |
| Dex-MA DS=18% | 161 ± 9 |
| Dex-MA DS=9% | 155 ± 7 |

Il n'est pas constaté d'altération des propriétés mécaniques des matériaux. Il est avantageux de noter que les modules de compression des matériaux DexMA séchés photo-réticulés sont bien supérieurs à 10 kPa, la valeur seuil pour percer la peau.

### Exemple 2

### Préparation d'un patch micro-aiguilles, MN couplé à une bioélectrode de travail selon l'invention.

### a) Préparation d'une bioélectrode de travail (WE) en nanotubes de carbone avec revêtement de médiateur électrochimique (le PLQ) et d'enzyme (la FADGDH).

66 mg de MWCNT ont été dispersés dans 66 ml de DMF sur un flacon de 100 ml, et mis dans un bain-marie sous sonication pendant 1h30. La suspension a été filtrée sur filtre PTFE à l'aide d'une pompe à vide, lavé à l'eau distillée et laissé 2h sous hotte. Après filtration, le « papier » de nanotubes de carbone résultant a été laissé sécher à température ambiante contre un autre filtre PTFE pendant 24h. Le « papier » de nanotubes de carbone a été délicatement détaché du papier filtre et découpé en électrodes individuelles de Ø = 4 ou 6 mm. Ensuite, les MWCNT ont été respectivement modifiés par un premier ajout de 9 et 20 µl d'une solution de PLQ (5 mM) dans un mélange d'acétone/H₂O (rapport volumique de 1 : 1), et laissés sécher pendant 10 minutes. Respectivement 13,5 et 30 µl d'une solution de FADGDH dans du tampon phosphate PB (10 mg.mL-1) ont été déposés sur la surface de l'électrode modifiée, et laissés sécher quelques heures. Enfin, le contact électrique a été réalisé à l'aide d'un fil métallique fixé à l'arrière de la bioélectrode avec de la pâte de carbone et laissé sécher pendant 2h. Le dos de l'électrode a été isolé avec de la pâte de silicone et laissé sécher pendant quelques heures avant utilisation.

### b) Préparation du moule en polydiméthylsiloxane, PDMS, pour le patch MN

Un moule maître a été fabriqué en aluminium par micro-fraisage. Le moule en aluminium était composé d'un réseau cylindrique de 7 mm avec 5 x 5 (25) micro-aiguilles en forme de crayons avec des hauteurs respectives de 800, 1000 et 1200 µm, une largeur à la base de 400 µm, et une distance bord à bord entre les aiguilles de 400 µm. Un moule inverse a été préparé en coulant un mélange de PDMS et de son agent de durcissement (rapport de 10 : 1) sur le moule principal, sous vide pour éliminer les bulles d'air, puis durci à 100 ° C pendant 2 h. Enfin, les moules maître et inverse ont été refroidis sous air pendant quelques heures et le moule PDMS a été délicatement détaché du moule en aluminium.

### c) Préparation du patch MN avec intégration de l'électrode de travail

Une solution de PBS contenant 20 % (p/p) de Dex-MA avec un DS de 9, 18, 37 ou 62 % et 1 % (p/p) de LAP comme photoinitiateur, a été préparée dans un flacon de 10 ml. 100 µl de solution ont été versés dans un moule PDMS. Le moule a été placé dans un support en aluminium relié à une pompe à vide, et une pression réduite (1-10 mbar) a été appliquée pendant 2h pour remplir les pointes du moule. L'électrode de travail, WE, préparée en a) a ensuite été intégrée dans le patch MN en insérant l'électrode à l'intérieur de la solution polymère de telle sorte que la surface de l'électrode soit proche de l'arrière des pointes, et l'arrière de l'électrode a été exposé pour permettre la connexion électrique 9. Le patch MN avec l'électrode, WE, intégrée a ensuite été laissé sécher pendant 24h. Le polymère a été réticulé par une première irradiation du dos du patch à 405 nm pendant 1 min (P = 75 mW/cm²), suivie du démoulage du dispositif et d'une dernière irradiation à 405 nm pendant 1 min sur la face des aiguilles du patch. Enfin, le raccordement électrique a été effectué par collage d'un fil d'Ag/Cu avec de la pâte de carbone a l'arrière de l'électrode.

### Exemple 3

### Test d'efficacité du dispositif MN avec l'électrode de travail à base de MWCNT intégrée pour l'électrooxydation et la détection du glucose

Le dispositif MN considéré est un patch micro-aiguilles Dex-MA (DS = 9%) réticulé intégrant une électrode de travail à base de MWCNT avec PLQ et FADGDH immobilisés et préparé selon l'exemple 2c).

Les voltammogrammes cycliques, représentés en figure 2 ont été enregistrés en l'absence et en présence de glucose dans une solution tampon phosphate préparée à 0,1 M, pH = 7,4 (PB) dans l'air, dans une cellule à 3 électrodes employant le dispositif MN comme électrode de travail, un fil de platine comme contre-électrode, et un fil d'argent chloré (Ag/AgCl) comme électrode de référence. En l'absence de glucose (ligne pointillée), on observe le signal redox du PLQ avec une activité redox typique de la quinone (couple redox bas potentiel dominant autour de E1/2 = -0,24 V vs pseudo-référence Ag/AgCl). En présence de glucose 0,1 M (ligne pleine), une forte augmentation du courant est observée avec un potentiel de déclenchement attractif faible d'environ -0,3 V par rapport à la pseudo-référence Ag/AgCl. Il ressort que le processus d'intégration de la bioélectrode dans la matrice d'hydrogel n'a pas perturbé de manière significative l'oxydation bioélectrocatalytique du glucose. Il a été vérifié cette même efficacité de l'oxydation bioélectrocatalytique avec des patchs micro-aiguilles de Dex-MA de DS de 18, 37 et 62 %.

### Exemple 4

### Application d'un dispositif selon l'invention pour une mesure en continu de la glycémie dans un fantôme de peau artificielle bicouche

Une surveillance continue du glucose a été réalisée avec l'électrode MN Dex-MA réticulé intégrée (DS = 37%) préparé selon l'exemple 2 dans un fantôme de peau artificielle bicouche tel que détaillé ci-dessus dans le chapitre *Matériels et méthodes.*

Les mesures ampérométriques ont été réalisées comme décrites également dans le chapitre *Matériel et Méthodes.* Les contre-électrodes et les électrodes de référence étaient ici insérées directement dans le fantôme de peau (pas d'intégration avec le patch MN). Les aiguilles du dispositif MN en Dex-MA (DS = 37%) réticulé intégrant une électrode de travail de type bioélectrode à base de MWCNTs recouverts par du PLQ et de la FADGDH tel que préparé selon l'exemple 2c) ont percé le fantôme de peau artificielle bicouche (24%) en utilisant une force de pénétration de 10 N, et une force constante d'environ 1 à 2 N pour maintenir les aiguilles à l'intérieur du derme artificiel.

Immédiatement après l'insertion et l'application concomitante de -0,1 V, le courant enregistré a augmenté jusqu'à environ 3 µA (Figure 3). En l'absence de glucose, le courant ne dépasse pas 0,1 µA. Le courant catalytique maximal a été atteint à environ 4 h. Une diminution du courant, typique de ces électrodes enzymatiques de glucose de 2e génération, a ensuite été obtenue jusqu'à environ 22 h où les performances de l'électrode ont diminué à moins de 2 % du maximum de courant catalytique.

### Exemple 5

### Dispositif pour la détection de glucose avec une bioélectrode à base de MWCNT sans hydrogel versus un dispositif selon l'invention avec hydrogel réticulé (DexMA 37%): Caractérisation en présence de différentes espèces interférentes dans l'ISF artificiel .

La mesure ampérométrique avec l'électrode de glucose considérée en exemple précédent, peut être réalisée à différents potentiels, le potentiel appliqué ayant un impact sur des facteurs tels que la contribution des interférences électrochimiques ainsi que la bioélectrocatalyse qui affecte les performances du capteur.

La figure 4 montre un chronoampérogramme enregistré à -0,1 V qui montre, premièrement, un signal de capteur de glucose (courant d'oxydation) après addition de 5 mmolL⁻¹ de glucose physiologiquement pertinent. Des ajouts successifs d'interférents potentiels ont ensuite été effectués. Le courant est resté pratiquement inchangé avec l'ajout d'acétaminophène, de cholestérol, d'urée, de lactate, de galactose et d'acide urique (UA) (2-7). L'acide urique et l'acétaminophène sont connus pour être oxydés à des potentiels qui interfèrent avec les capteurs de glucose et sont donc connus comme des interférents électrochimiques courants. En revanche, le signal de l'éléctrode de glucose (courant d'oxydation) change de manière significative avec l'ajout d'acide ascorbique (AA) (8) correspondant à l'oxydation électrochimique du composé au potentiel de travail de -0,1 V par rapport à la pseudo référence Ag/AgCl. Néanmoins, ce courant n'implique qu'un taux d'interférence de 6,8%, ce qui est très acceptable. Enfin, un deuxième ajout de 5 mmol de glucose L-1 a été effectué (1) qui a entraîné à nouveau une augmentation du courant, montrant la robustesse du dispositif biocapteur à base de MWCNT à la présence d'espèces interférentes. Toutefois, ce courant est inférieur au courant issu du premier ajout de glucose.

La même expérience a été réalisée sur des dispositifs présentant un revêtement à base de Dex-MA DS = 37% réticulé conforme à l'invention. Similairement aux dispositifs sans hydrogel, une augmentation du courant est obtenu lors du premier ajout du glucose, suivi par un courant pratiquement inchangé avec l'ajout d'acétaminophène, de cholestérol, d'urée, de lactate, de galactose et d'acide urique (UA) (2-7), une oxydation de l'acide ascorbique est observée lors de son ajout en raison de son oxydation à ce potentiel de travail, impliquant au taux d'interférence de seulement 4,9%, ce qui montre l'effet bénéfique de l'hydrogel vis-à-vis des espèces interférentes. Enfin, on observe une dernière augmentation du courant suite au dernier ajout de glucose, augmentation dont la valeur est très proche de celle obtenue lors du premier ajout, montrant l'effet protecteur et stabilisant de l'hydrogel aux espèces interférentes vis-à-vis du dispositif.

### Exemple 6

### Caractérisation de la stabilité d'un patch MN avec une bioélectrode intégrée selon l'invention versus une électrode immobilisée en dos de patch.

Une solution de PBS contenant 20 % (p/p) de Dex-MA avec un DS de 37% et 1 % (p/p) de LAP comme photoinitiateur, a été préparée dans un flacon de 10 ml. La solution a été coulée dans le moule PDMS préparé en exemple 1. Le moule a été placé dans un support en aluminium relié à une pompe à vide, et une pression réduite (1-10 mbar) a été appliquée pendant 24h pour remplir les pointes du moule et sécher le polymère. Le polymère sec a ensuite été réticulé pendant sous UV-vis à 405 nm pendant 1 min (P = 75 mW/cm2). L'électrode à base de MWCNT (préparée en exemple 2) a ensuite été intégrée en déposant une couche de polymère contenant 20 % (p/p) de Dex-MA avec un DS de 37% et 1% (p/p) de LAP sur l'électrode et en collant l'électrode au dos du patch. Le patch MN ainsi obtenu a ensuite été exposé de nouveau aux UV-vis à 405 nm pendant 1 min afin de réticuler la couche collante.

Ce dispositif non conforme à l'invention et celui conforme à l'invention préparé à l'aide d'un Dex-MA de DS de 37%, en exemple 2, ont ensuite été analysés par chronoampérométrie à -0,1V en immergeant la partie hydrogel du dispositif dans une solution de PB sous agitation pendant 1h min, suivi par un ajout de 5 mM de glucose. Toutefois, après 24h, aucun courant catalytique n'a été observé avec le patch MN non conforme à l'invention. Un décollement partiel de l'électrode a également été observé.

## Revendications

1. Dispositif utile pour des mesures électrochimiques transdermiques, ledit dispositif comportant au moins un support polymérique (1) ayant une surface dédiée à un contact avec une peau (2), au moins un réseau de micro-aiguilles polymériques (3) solidaires dudit support (1) et dépassant vers l'extérieur de ladite surface du support (1) dédiée au contact avec ladite peau (2), et au moins une bioélectrode (10), comprenant au moins une espèce biologiquement active, notamment une enzyme, immobilisée à la surface d'un matériau conducteur, lesdites micro-aiguilles (3) et au moins la surface de contact dudit support (1) avec ladite peau (2) étant formées d'un hydrogel réticulé, biocompatible, non conducteur électronique à l'état sec et conducteur électrolytique au contact d'un fluide aqueux, et ladite bioélectrode (10) étant disposée au contact de l'hydrogel dédié à gonfler au contact dudit fluide aqueux, étant dénuée de contact direct avec la peau (2) et **caractérisé en ce que** ladite bioélectrode est poreuse et au moins partiellement interpénétrée d'hydrogel réticulé.

2. Dispositif selon la revendication précédente dans lequel ladite ou au moins une bioélectrode (10) est intégrée dans l'hydrogel réticulé constitutif du support (1) qui est en contact direct avec l'arrière des microaiguilles (3).

3. Dispositif selon l'une quelconque des revendications précédentes **caractérisé en ce que** ledit hydrogel est dénué de constituant métallique et de polymère conducteur électronique.

4. Dispositif selon l'une quelconque des revendications précédentes **caractérisé en ce que** ledit hydrogel est dénué d'espèce biologiquement active autre que celle(s) immobilisée(s) sur la ou lesdites bioélectrode(s) (10).

5. Dispositif selon l'une quelconque des revendications précédentes **caractérisé en ce que** lesdites micro-aiguilles polymériques (3) ne sont pas creuses.

6. Dispositif selon l'une quelconque des revendications précédentes dans lequel ladite espèce biologiquement active est une enzyme, le cas échéant combinée à une molécule qui facilite le transfert des électrons entre l'enzyme et ladite électrode 10.

7. Dispositif selon l'une quelconque des revendications précédentes dans lequel la bioélectrode (10) est formée d'un matériau conducteur nano-structuré ou micro-structuré et en particulier est à base de nanotubes de carbone et plus particulièrement est à base de nanotubes de carbone multiparois, MWCNT.

8. Dispositif selon l'une quelconque des revendications précédentes **caractérisé en ce qu'**il comprend au moins une bioélectrode enzymatique et, en particulier, dédiée à interagir avec le glucose et qui associe l'enzyme FAD-GDH au médiateur redox phénanthroline quinone, PLQ, adsorbée physiquement au niveau d'une électrode en nanotubes de carbone multiparois, MWCNT.

9. Dispositif selon l'une quelconque des revendications précédentes dans lequel ledit hydrogel est obtenu par réticulation d'un ou plusieurs biopolymères avec le cas échéant d'un ou plusieurs polymères synthétiques, le cas échéant modifiés chimiquement pour être réticulables, ou d'un de leurs mélanges.

10. Dispositif selon la revendication précédente dans lequel ledit biopolymère est choisi parmi les polyhydroxyacides, tels que par exemple l'acide polyglycolique, PGA, et l'acide polylactique, PLA, également connu sous le nom de polylactide ; les polysaccharides, tels que par exemple la cellulose, la chitine et l'amidon et des protéines ou des peptides, tels que par exemple le collagène, la gélatine, les élastines, les fibroïnes, la zéine de maïs, la soie de diverses espèces de vers à soie et la kératine et leurs dérivés.

11. Dispositif selon la revendication précédente dans lequel ledit biopolymère est ou dérive d'au moins un polymère choisi parmi les alginates, l'acide hyaluronique, la carboxyméthylcellulose, le chitosan, le dextran, et leurs dérivés, et en particulier le dextran.

12. Dispositif selon l'une quelconque des revendications précédentes dans lequel ledit hydrogel dérive de la réticulation d'au moins un polymère de dextran modifié par des motifs choisis parmi les acrylate, méthacrylate, alcényle et alcynyle et en particulier un polymère de dextran modifié par des motifs méthacrylate, Dex-MA, et de DS variant de 5 à 65 %.

13. Dispositif selon l'une quelconque des revendications précédentes se présentant sous la forme d'un patch transdermique micro-aiguilles, MN.

14. Dispositif selon l'une quelconque des revendications précédentes comportant au moins une électrode annexe, distincte ou non d'une bioélectrode, notamment choisie parmi une électrode de référence, une contre-électrode, ou une électrode hybride de référence et contre-électrode.

15. Dispositif selon l'une quelconque des revendications précédentes comportant un dispositif de détection électrochimique attaché à ladite ou auxdites électrodes via des connections électriques.

16. Procédé de préparation d'un dispositif selon l'une quelconque des revendications précédentes par micromoulage et comprenant au moins une étape de réticulation d'au moins un biopolymère pour former ledit hydrogel réticulé **caractérisé en ce qu'**au moins ladite ou une bioélectrode poreuse dudit dispositif est intégrée par mise en contact avec ledit biopolymère avant ou simultanément à sa réticulation.

17. Utilisation d'un dispositif selon l'une quelconque des revendications 1 à 15 pour des mesures électrochimiques transdermiques, en particulier pour la caractérisation d'au moins un analyte dans un liquide interstitiel.

18. Utilisation selon la revendication 17 pour la détection et/ou le dosage d'au moins un analyte dans un liquide interstitiel, notamment d'une manière non invasive, comprenant au moins la mise en contact des micro-aiguilles (3) d'un dispositif selon l'une quelconque des revendications 1 à 15 avec ledit liquide interstitiel dans des conditions propices au gonflement dudit hydrogel constitutif des micro-aiguilles (3) par ce liquide et à la diffusion de ce liquide par différence de pression osmotique et/ou capillarité jusqu'à la ou les bioélectrode(s) (10) dudit dispositif.

## Patentansprüche

1. Vorrichtung, die für transdermale elektrochemische Messungen nützlich ist, die Vorrichtung aufweisend mindestens einen polymerischen Träger (1) mit einer zu einem Kontakt mit einer Haut (2) bestimmten Oberfläche, mindestens ein Gitter aus polymerischen Mikronadeln (3), die mit dem Träger (1) fest verbunden sind und von der zum Kontakt mit der Haut (2) bestimmten Oberfläche des Trägers (1) nach außen abragen, und mindestens eine Bioelektrode (10), die mindestens eine biologisch aktive Spezies, insbesondere ein Enzym, umfasst, die an der Oberfläche eines leitfähigen Materials immobilisiert ist, wobei die Mikronadeln (3) und mindestens die Kontaktfläche des Trägers (1) mit der Haut (2) aus einem biokompatiblen, im trockenen Zustand nicht elektronisch leitfähigen und beim Kontakt mit einer wässrigen Flüssigkeit elektrolytisch leitfähigen, vernetzten Hydrogel gebildet sind, und wobei die Bioelektrode (10) in Kontakt mit dem Hydrogel angeordnet ist, das dazu bestimmt ist, beim Kontakt mit der wässrigen Flüssigkeit aufzuquellen, wobei sie keinen direkten Kontakt mit der Haut (2) hat, und **dadurch gekennzeichnet, dass** die Bioelektrode porös ist und mindestens teilweise von dem vernetzten Hydrogel durchdrungen wird.

2. Vorrichtung nach dem vorhergehenden Anspruch, wobei die oder mindestens eine Bioelektrode (10) in das vernetzte Hydrogel integriert ist, aus dem der Träger (1) besteht, der in direktem Kontakt mit der Rückseite der Mikronadeln (3) ist.

3. Vorrichtung nach einem der vorhergehenden Ansprüche, **dadurch gekennzeichnet, dass** das Hydrogel keinen metallischen Bestandteil und kein elektronisch leitfähiges Polymer aufweist.

4. Vorrichtung nach einem der vorhergehenden Ansprüche, **dadurch gekennzeichnet, dass** das Hydrogel keine andere biologisch aktive Spezies als die auf der oder den Bioelektrode(n) (10) immobilisierte(n) aufweist.

5. Vorrichtung nach einem der vorhergehenden Ansprüche, **dadurch gekennzeichnet, dass** die polymerschen Mikronadeln (3) nicht hohl sind.

6. Vorrichtung nach einem der vorhergehenden Ansprüche, wobei die biologisch aktive Spezies ein Enzym ist, gegebenenfalls kombiniert mit einem Molekül, das den Transfer der Elektronen zwischen dem Enzym und der Elektrode 10 erleichtert.

7. Vorrichtung nach einem der vorhergehenden Ansprüche, wobei die Bioelektrode (10) aus einem nanostrukturierten oder mikrostrukturierten leitfähigen Material gebildet ist und insbesondere auf Kohlenstoffnanoröhren basiert und im Besonderen auf mehrwandigen Kohlenstoffnanoröhren, MWCNT, basiert.

8. Vorrichtung nach einem der vorhergehenden Ansprüche, **dadurch gekennzeichnet, dass** sie mindestens eine Bioelektrode umfasst, die enzymatisch ist und insbesondere dazu bestimmt ist, mit Glucose zu interagieren, und die das Enzym FAD-GDH mit dem Redoxmediator Phenanthrolinchinon, PLQ, kombiniert, der physikalisch an einer Elektrode aus mehrwandigen Kohlenstoffnanoröhren, MWCNT, adsorbiert wird.

9. Vorrichtung nach einem der vorhergehenden Ansprüche, wobei das Hydrogel durch Vernetzen von einem oder mehreren Biopolymeren mit gegebenenfalls einem oder mehreren synthetischen Polymeren, die gegebenenfalls chemisch modifiziert sind, um vernetzbar zu sein, oder einer ihrer Mischungen erhalten wird.

10. Vorrichtung nach dem vorhergehenden Anspruch, wobei das Biopolymer ausgewählt ist aus den Polyhydroxysäuren, wie zum Beispiel Polyglykolsäure, PGA, und Polymilchsäure, PLA, auch bekannt unter dem Namen Polylactid; den Polysacchariden, wie zum Beispiel Cellulose, Chitin und Stärke, und Proteinen oder Peptiden, wie zum Beispiel Kollagen, Gelatine, Elastine, Fibroine, Maiszein, Seide von verschiedenen Seidenraupenspezies und Keratin und ihren Derivaten.

11. Vorrichtung nach dem vorhergehenden Anspruch, wobei das Biopolymer mindestens ein Polymer ist oder aus mindestens einem Polymer hervorgegangen ist, das aus den Alginaten, Hyaluronsäure, Carboxymethylcellulose, Chitosan, Dextran und ihren Derivaten und insbesondere Dextran ausgewählt ist.

12. Vorrichtung nach einem der vorhergehenden Ansprüche, wobei das Hydrogel aus der Vernetzung mindestens eines Dextranpolymers hervorgegangen ist, das durch Einheiten modifiziert ist, die aus Acrylat, Methacrylat, Alkenyl und Alkynyl ausgewählt sind, und insbesondere eines durch Dextranpolymers, das durch Methacrylat-Einheiten modifiziert ist, Dex-MA, und einen DS von 5 % bis 65 % aufweist.

13. Vorrichtung nach einem der vorhergehenden Ansprüche, die in Form eines transdermalen Mikronadelpflasters, MN, vorliegt.

14. Vorrichtung nach einem der vorhergehenden Ansprüche, aufweisend mindestens eine Zusatzelektrode, die von einer Bioelektrode verschieden ist oder nicht und die insbesondere aus einer Referenzelektrode, einer Gegenelektrode oder einer hybriden Referenz- und Gegenelektrode ausgewählt ist.

15. Vorrichtung nach einem der vorhergehenden Ansprüche, aufweisend eine elektrochemische Detektionsvorrichtung, die an die Elektrode(n) über elektrische Anschlüsse angebunden ist.

16. Verfahren zum Herstellen einer Vorrichtung nach einem der vorhergehenden Ansprüche durch Mikroabformung und umfassend mindestens einen Schritt des Vernetzens mindestens eines Biopolymers, um das vernetzte Hydrogel zu bilden, **dadurch gekennzeichnet, dass** mindestens die oder eine poröse Bioelektrode der Vorrichtung durch Inkontaktbringen mit dem Biopolymer vor oder gleichzeitig mit seiner Vernetzung integriert wird.

17. Verwendung einer Vorrichtung nach einem der Ansprüche 1 bis 15 für transdermale elektrochemische Messungen, insbesondere zur Charakterisierung mindestens eines Analyten in einer interstitiellen Flüssigkeit.

18. Verwendung nach Anspruch 17 zur Detektion und/oder zur Dosierung mindestens eines Analyten in einer interstitiellen Flüssigkeit, insbesondere auf nicht invasive Weise, umfassend mindestens das Inkontaktbringen der Mikronadeln (3) einer Vorrichtung nach einem der Ansprüche 1 bis 15 mit der interstitiellen Flüssigkeit unter Bedingungen, die für das Aufquellen des Hydrogels, aus dem die Mikronadeln (3) bestehen, durch diese Flüssigkeit und für die Diffusion dieser Flüssigkeit durch eine osmotische Druckdifferenz und/oder Kapillarität bis zu der oder den Bioelektrode(n) (10) der Vorrichtung günstig sind.

## Claims

1. Device of use for transdermal electrochemical measurements, said device comprising at least one polymeric support (1) having a surface provided for contact with skin (2), at least one array of polymeric microneedles (3) integral with said support (1) and projecting outward from said surface of the support (1) provided for contact with said skin (2), and at least one bioelectrode (10) comprising at least one biologically active species, in particular an enzyme, immobilized on the surface of a conductive material, said microneedles (3) and at least the surface of contact of said support (1) with said skin (2) being formed of a biocompatible crosslinked hydrogel, non-electron-conductive in the dry state and electrolyte-conductive upon contact with an aqueous fluid, and said bioelectrode (10) being arranged in contact with the hydrogel provided to swell upon contact with said aqueous fluid, being free of direct contact with the skin (2) and **characterized in that** said bioelectrode is porous and at least partially interpenetrated by crosslinked hydrogel.

2. Device according to the preceding claim, in which said or at least one bioelectrode (10) is integrated in the crosslinked hydrogel constituting the support (1) which is in direct contact with the rear of the microneedles (3).

3. Device according to either one of the preceding claims, **characterized in that** said hydrogel is free of any metallic constituent and electron-conductive polymer.

4. Device according to any one of the preceding claims, **characterized in that** said hydrogel is free of any biologically active species other than that/those immobilized on the or said bioelectrode(s) (10).

5. Device according to any one of the preceding claims, **characterized in that** said polymeric microneedles (3) are not hollow.

6. Device according to any one of the preceding claims, in which said biologically active species is an enzyme, optionally combined with a molecule that facilitates electron transfer between the enzyme and said electrode 10.

7. Device according to any one of the preceding claims, in which the bioelectrode (10) is formed of a nano-structured or micro-structured conductive material and in particular is based on carbon nanotubes and more particularly is based on multi-walled carbon nanotubes, MWCNT.

8. Device according to any one of the preceding claims, **characterized in that** it comprises at least one enzymatic bioelectrode which is provided in particular to interact with glucose and which associates the FAD-GDH enzyme with the redox mediator phenanthroline quinone, PLQ, physically adsorbed at the level of an electrode formed of multi-walled carbon nanotubes, MWCNT.

9. Device according to any one of the preceding claims, in which said hydrogel is obtained by crosslinking one or more biopolymers with, where appropriate, one or more synthetic polymers, where appropriate chemically modified to be crosslinkable, or a mixture thereof.

10. Device according to the preceding claim, in which said biopolymer is chosen from polyhydroxy acids, for example polyglycolic acid, PGA, and polylactic acid, PLA, also known as polylactide; polysaccharides, for example cellulose, chitin and starch, and proteins or peptides, for example collagen, gelatin, elastins, fibroins, maize zein, the silk of various species of silkworms and keratin and their derivatives.

11. Device according to the preceding claim, in which said biopolymer is or derives from at least one polymer chosen from alginates, hyaluronic acid, carboxymethylcellulose, chitosan, dextran, and derivatives thereof, and in particular dextran.

12. Device according to any one of the preceding claims, in which said hydrogel derives from the crosslinking of at least one dextran polymer modified by units chosen from acrylate, methacrylate, alkenyl and alkynyl, and in particular a dextran polymer modified by methacrylate units, Dex-MA, and of DS varying from 5 to 65%.

13. Device according to any one of the preceding claims, in the form of a transdermal microneedle, MN, patch.

14. Device according to any one of the preceding claims, comprising at least one auxiliary electrode, distinct or not from a bioelectrode, in particular chosen from a reference electrode, a counter-electrode, or a hybrid reference electrode and counter-electrode.

15. Device according to any one of the preceding claims, comprising an electrochemical detection device attached to said electrode(s) via electrical connections.

16. Method for preparing a device according to any one of the preceding claims by micro-moulding and comprising at least one step of crosslinking at least one biopolymer in order to form said crosslinked hydrogel, **characterized in that** at least said or a porous bioelectrode of said device is integrated by being placed in contact with said biopolymer prior to or simultaneously with its crosslinking.

17. Use of a device according to any one of Claims 1 to 15 for transdermal electrochemical measurements, in particular for characterizing at least one analyte in an interstitial fluid.

18. Use according to Claim 17 for detecting and/or assaying at least one analyte in an interstitial fluid, in particular in a non-invasive manner, comprising at least placing the microneedles (3) of a device according to any one of Claims 1 to 15 in contact with said interstitial fluid under conditions that are conducive to the swelling of said constituent hydrogel of the microneedles (3) by this fluid and to the diffusion of this fluid, by a difference in osmotic pressure and/or capillarity, as far as the bioelectrode(s) (10) of said device.
